# FASCICULE DE BREVET EUROPEEN

(11) **EP 1 867 352 B1**
(45) Date de publication et mention de la délivrance du brevet: **15.07.2009**
(21) Numéro de dépôt: 07290725.6
(22) Date de dépôt: 11.06.2007
(51) Int. Cl.: A61M 1/10

(54) **Prothèse cardiaque monobloc implantable en position anatomique**
Einstückige, in anatomischer Lage implantierbare Herzprothese
Anatomically implantable heart prosthesis in one piece

(30) Priorité: 15.06.2006 FR 0605333
(43) Date de publication de la demande: 19.12.2007
(73) Titulaire: Carmat, 75008 Paris (FR)
(72) Inventeur: Grimmé, Marc, 75019 Paris (FR); Koechler, Maurice, 91400 Orsay (FR); Parquet, Jean-Marc, 95330 Domont (FR); Carpentier, Alain, 75116 Paris (FR)
(74) Mandataire: Bonnetat, Christian

(56) Documents cités:
- FR-A1- 2 585 250
- US-A- 5 135 539
- US-B1- 6 342 072
- SHAH A S: "INTRAOPERATIVE DETERMINATION OF MEDIASTINAL CONSTRAINTS FOR A TOTAL ARTIFICIAL HEART" ASAIO TRANSACTIONS, HARPER AND ROW PUBLISHERS, HAGERSTOWN, MD, US, vol. 37, no. 2, 1 avril 1991 (1991-04-01), pages 76-79, XP000215535

## Description

La présente invention concerne une prothèse cardiaque monobloc implantable en position anatomique.

Par le document US-5 135 539, on connaît déjà une prothèse cardiaque, implantable dans la cavité péricardique d'un patient et apte à remplacer les ventricules gauche et droit naturels dudit patient, après ablation de ceux-ci. Cette prothèse cardiaque comporte un corps rigide dans lequel sont agencés des ventricules gauche et droit artificiels, chacun de ces ventricules artificiels comprenant une membrane souple :
- qui est apte à battre sous l'action d'un fluide hydraulique, et
- qui est disposée dans une cavité que ladite membrane partage de façon étanche en deux chambres dont l'une est destinée à la circulation du sang et dont l'autre est emplie dudit fluide hydraulique.

Par ailleurs, la chambre à fluide hydraulique de chacun desdits ventricules artificiels est reliée à un actionneur hydraulique individuel, lui-même en communication avec un sac étanche entourant la prothèse et contenant ledit fluide hydraulique. La chambre à sang du ventricule artificiel gauche comporte un orifice de raccord à l'oreillette naturelle gauche et des moyens de raccord à l'aorte, tandis que la chambre à sang du ventricule artificiel droit comporte un orifice de raccord à l'oreillette naturelle droite et des moyens de raccord à l'artère pulmonaire, les axes desdits orifices de raccord aux oreillettes naturelles étant coplanaires et lesdits ventricules artificiels présentant, parallèlement au plan desdits axes desdits orifices, des directions générales disposées en V, de façon que lesdits ventricules se rapprochent l'un de l'autre en s'éloignant desdits orifices de raccord aux oreillettes naturelles.

Dans la réalisation du document US-5 135 539, les ventricules artificiels de ladite prothèse cardiaque sont disposés de façon rigoureusement symétrique par rapport à un plan médian, avec leurs actionneurs hydrauliques individuels opposés faisant latéralement saillie par rapport audit corps rigide. Il en résulte que l'encombrement de ladite prothèse n'est pas optimal et qu'il peut en résulter des difficultés d'implantation dans la cavité thoracique et péricardique de nombreux patients.

La présente invention a pour objet de remédier à cet inconvénient. A cette fin, selon l'invention, la prothèse cardiaque du type rappelé ci-dessus est remarquable :
- en ce que lesdits axes desdits orifices de raccord aux oreillettes naturelles sont au moins approximativement parallèles ;
- en ce que ladite disposition en V des ventricules gauche et droit artificiels est asymétrique par rapport auxdits axes, l'angle formé entre ladite direction générale du ventricule artificiel droit et l'axe dudit orifice de raccord à l'oreillette naturelle droite étant plus grand que l'angle formé entre ladite direction générale du ventricule artificiel gauche et l'axe dudit orifice de raccord à l'oreillette naturelle gauche ; et
- en ce que lesdits actionneurs hydrauliques individuels, associés respectivement auxdits ventricules gauche et droit artificiels, sont disposés proches l'un de l'autre, du côté dudit ventricule artificiel gauche.

Ainsi, grâce à la présente invention, le positionnement déporté desdits actionneurs hydrauliques individuels et la disposition en V asymétrique desdits ventricules artificiels permettent de positionner les compartiments sanguins en regard des oreillettes naturelles correspondantes tout en donnant à ladite prothèse une forme et un volume proches de la forme et du volume anatomiques de la cavité péricardique, permettant donc de loger ladite prothèse dans cette dernière.

Dans le cas où la prothèse conforme à la présente invention est destinée à un adulte, la distance entre lesdits axes parallèles peut être au moins approximativement égale à 45 mm.

Quant aux actionneurs hydrauliques individuels, ils peuvent être disposés n'importe où du côté du ventricule artificiel gauche, notamment au voisinage de la pointe dudit V.

Afin d'améliorer encore les effets bénéfiques apportés par l'invention, il est avantageux que les dimensions desdits ventricules artificiels, parallèlement auxdites directions générales, soient plus petites que les dimensions desdits ventricules perpendiculairement auxdites directions générales et que les directions générales des deux ventricules artificiels fassent entre elles un angle au moins approximativement égal à 80°.

Ainsi, on gagne encore en encombrement parallèlement à l'axe antéro-postérieur du thorax.

Dans le cas usuel où chacun desdits ventricules artificiels présente la forme de deux dômes opposés par rapport à une base commune, il est alors avantageux que ladite base commune présente la forme d'une ellipse, dont le petit axe est au moins sensiblement parallèle à ladite direction générale correspondante.

Dans le cas particulier où la prothèse est destinée à un adulte et où chaque ventricule artificiel doit présenter un volume de l'ordre de 70 cm³, les longueurs du petit axe et du grand axe de ladite base elliptique sont de préférence au moins approximativement égales à 64 mm et 87 mm, respectivement. La distance entre les sommets des deux dômes d'un ventricule est alors au moins approximativement égale à 30 mm.

D'une manière générale, la disposition asymétrique et imbriquée des ventricules et des actionneurs hydrauliques, qui constitue une des caractéristiques originales de ladite prothèse cardiaque, permet d'en réduire au maximum l'encombrement, facilitant ainsi son logement dans la cavité péricardique.

Dans l'exemple de réalisation ci-dessus, dans lequel lesdites directions générales des deux ventricules artificiels font entre elles un angle au moins approximativement égal à 80°, il est avantageux que l'angle formé entre la direction générale du ventricule artificiel droit et l'axe dudit orifice de raccord à l'oreillette naturelle droite soit au moins approximativement égal à 50° et que l'angle formé entre la direction générale du ventricule artificiel gauche et l'axe dudit orifice de raccord à l'oreillette naturelle gauche soit au moins approximativement égal à 30°.

Du fait de la disposition desdits actionneurs hydrauliques individuels au voisinage du ventricule artificiel gauche, l'actionneur hydraulique individuel associé au ventricule droit artificiel est éloigné de ce dernier. Aussi, selon la présente invention, on prévoit un conduit, extérieur audit corps rigide, pour relier ledit ventricule droit artificiel à l'actionneur hydraulique individuel associé.

De façon connue, chaque ventricule artificiel peut être constitué, d'une part, par un évidement en forme de dôme pratiqué dans ledit corps rigide de façon que le bord de cet évidement forme ladite base commune et, d'autre part, par un couvercle, également en forme de dôme, apte à être rapporté à ladite base commune en fixant la membrane correspondante à cette dernière. Dans cette disposition, chaque actionneur hydraulique individuel communique avec le ventricule correspondant à travers le couvercle associé. Aussi, dans ce cas, l'actionneur hydraulique associé au ventricule droit artificiel est relié au couvercle de ce dernier par ledit conduit extérieur audit corps rigide.

De plus, ladite prothèse cardiaque conforme à la présente invention peut comporter :
- un sac souple entourant, de façon ample et étanche, au moins une partie dudit corps rigide en enfermant lesdits actionneurs hydrauliques et l'électronique de commande, de traitement des signaux et de communication, ledit sac étant rempli dudit fluide hydraulique et servant de bâche au circuit hydraulique desdits actionneurs. Ledit sac de par son étendue très large est animé de battements de faible amplitude, ce qui évite ainsi le besoin d'une chambre de compliance à distance de la prothèse. De plus, entourant les actionneurs et l'électronique de commande, ledit sac protège cette dernière et facilite les échanges thermiques entre actionneurs, chambres ventriculaires et tissus voisins; et
- une paroi rigide ajourée enveloppante, solidaire dudit corps rigide et disposée entre celui-ci et ledit sac souple, de façon que ledit sac souple ne soit pas aspiré par les actionneurs lors du remplissage des ventricules.

Entre ladite paroi rigide ajourée et ledit sac souple, on ménage avantageusement un volume de débattement pour ce dernier. Un tel volume de débattement peut être au moins approximativement égal au double du volume de la chambre à fluide hydraulique d'un desdits ventricules artificiels et réparti sur toute la surface de la paroi rigide.

Les figures du dessin annexé feront bien comprendre comment l'invention peut être réalisée. Sur ces figures, des références identiques désignent des éléments semblables.

Les figures 1 et 2 illustrent, en coupe transversale schématique du thorax d'un patient, le processus préparatoire à l'implantation de la prothèse cardiaque selon l'invention.

La figure 3 montre schématiquement un exemple de réalisation de la prothèse cardiaque de l'invention.

La figure 4 illustre, en coupe semblable aux figures 1 et 2, l'implantation de la prothèse de la figure 3.

La figure 5 illustre, en vue semblable à la figure 3, une variante de réalisation de la prothèse cardiaque conforme à la présente invention.

La figure 6 illustre la forme elliptique des ventricules artificiels de la prothèse conforme à la présente invention.

La figure 7 est une vue en perspective de la prothèse de l'invention, sans son enveloppe constituée d'un sac souple étanche.

La figure 8 est une vue de dessus de la prothèse de l'invention.

La figure 9 est une vue en perspective de la prothèse de l'invention, après élimination dudit sac souple étanche et de la paroi ajourée enveloppante.

La figure 10 est une vue en perspective montrant les actionneurs hydrauliques et les couvercles des ventricules artificiels.

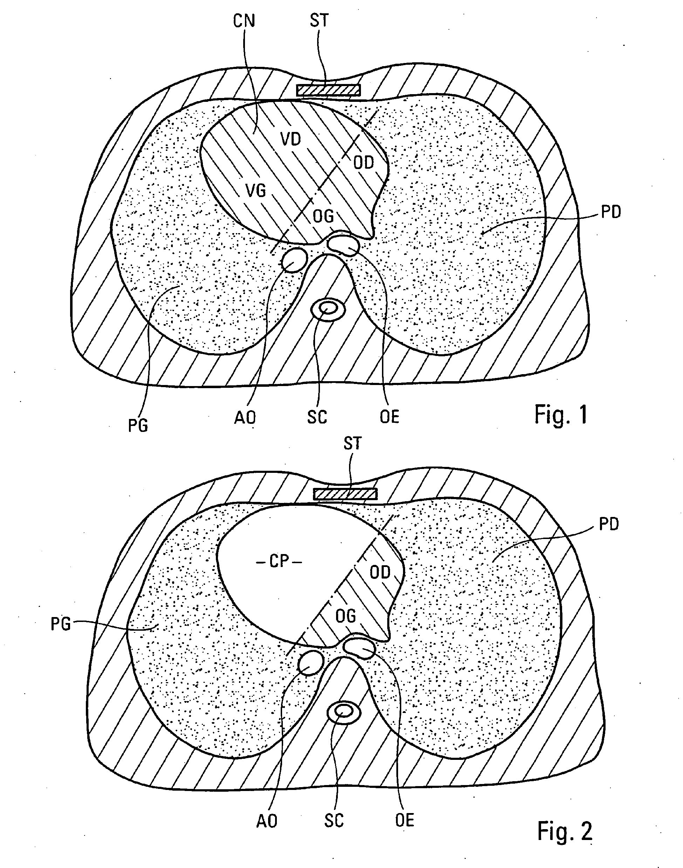

Sur la coupe transversale de thorax schématique de la figure 1, on a représenté le poumon gauche PG, le poumon droit PD, le sternum ST, l'aorte AO, la moelle épinière SC, l'oesophage OE et le coeur naturel CN d'un patient. Sur ce coeur naturel, on a indiqué l'oreillette gauche OG, l'oreillette droite OD, le ventricule gauche VG et le ventricule droit VD.

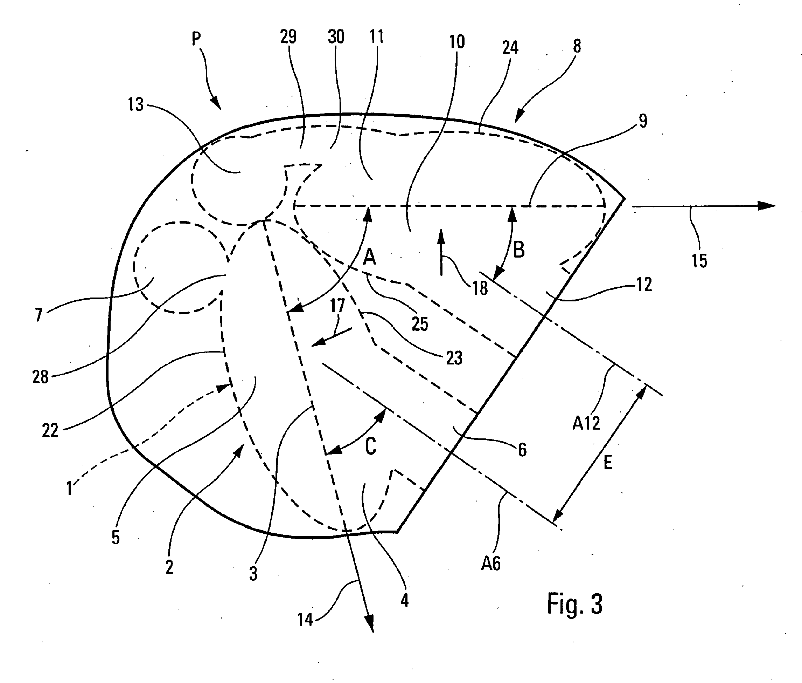

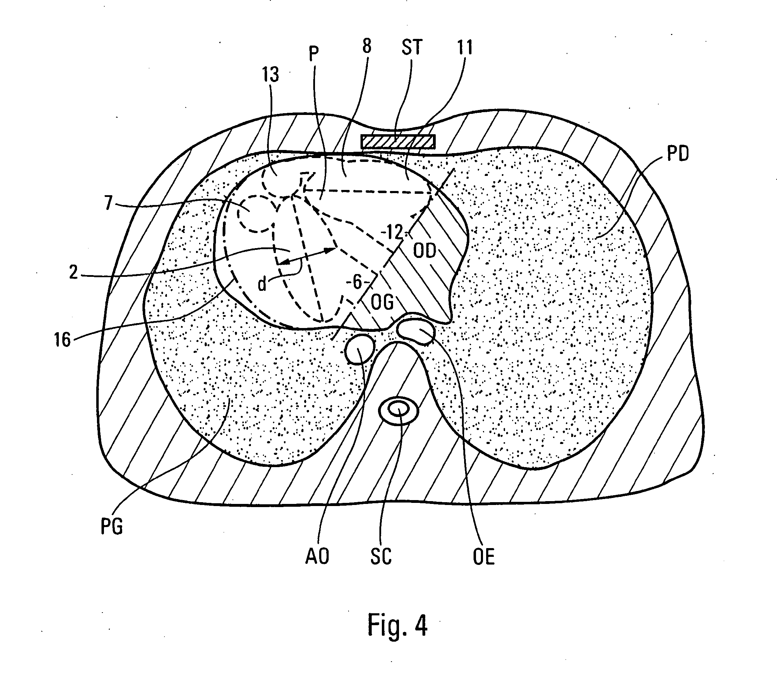

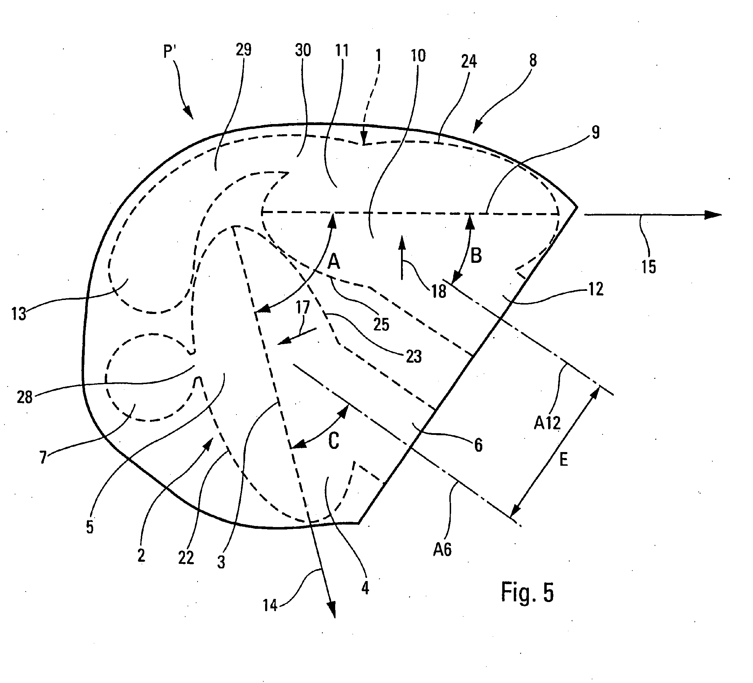

Les prothèses cardiaques P et P' conformes à la présente invention, représentées schématiquement à plus grande échelle sur les figures 3 et 5 respectivement, sont destinées à remplacer les ventricules gauche et droit VG et VD, après ablation de ceux-ci, comme cela est illustré schématiquement par la figure 2. Pour ce faire, les prothèses cardiaques P et P' doivent pouvoir être logées dans la partie de la cavité péricardique CP, laissée libre par l'élimination desdits ventricules VG et VD (voir les figures 2 et 4).

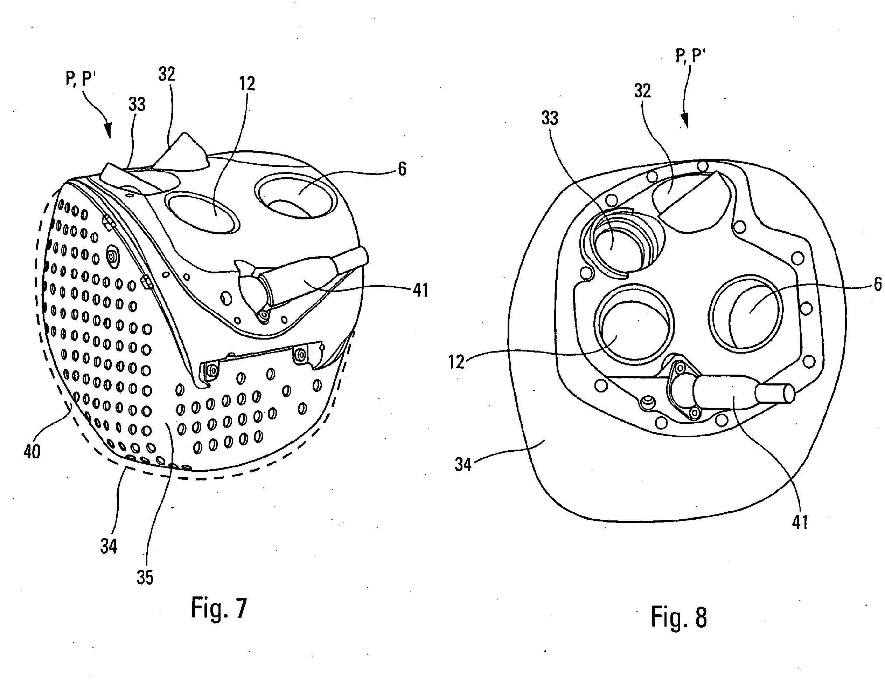

Comme cela est montré schématiquement par les figures 3, 5 et 9, les prothèses cardiaques P et P' comportent un corps rigide 1, dans lequel sont agencés :
- un ventricule gauche artificiel 2, comprenant une membrane souple 3 qui sépare de façon étanche ledit ventricule artificiel 2 en une chambre 4 pour la circulation du sang et en une chambre 5 pour un fluide hydraulique, ladite chambre à sang 4 comportant un orifice de raccord 6 à l'oreillette naturelle gauche OG et des moyens 33 de raccord à l'aorte AO (non visibles sur la figure 3, mais visibles sur la figure 8) ;
- un actionneur hydraulique 7, par exemple du type moto-pompe volumétrique, en communication avec la chambre 5 à fluide hydraulique du ventricule gauche artificiel 2 par un passage 28 ;
- un ventricule droit artificiel 8, comprenant une membrane souple 9 qui sépare de façon étanche ledit ventricule artificiel 8 en une chambre 10 pour la circulation du sang et en une chambre 11 pour un fluide hydraulique, ladite chambre à sang 10 comportant un orifice de raccord 12 à l'oreillette naturelle droite OD et des moyens 32 de raccord à l'artère pulmonaire (non visibles sur la figure 3, mais visibles sur la figure 8) ; et
- un actionneur hydraulique 13, par exemple également du type moto-pompe volumétrique, en communication avec la chambre 11 à fluide hydraulique du ventricule droit artificiel 8 par un conduit 29.

L'axe A6 de l'orifice de raccord 6 et l'axe A12 de l'orifice de raccord 12 sont au moins sensiblement parallèles, leur distance E étant au moins approximativement égale à 45 mm, dans le cas où la prothèse cardiaque P est destinée à un adulte.

De plus, comme cela est également montré de façon schématique par la figure 3 :
- les directions générales respectives 14 et 15 des ventricules artificiels gauche 2 et droit 8, parallèlement au plan des axes A6 et A12, sont convergentes, en faisant entre elles un angle A, par exemple de l'ordre de 80°, de sorte que lesdits ventricules sont disposés en V et se rapprochent l'un de l'autre en s'éloignant des orifices de raccord 6 et 12 à l'oreillette gauche OG et à l'oreillette droite OD, respectivement ;
- la disposition en V des ventricules artificiels gauche 2 et droit 8 est asymétrique par rapport aux axes A6 et A12, l'angle B formé entre la direction générale 15 du ventricule artificiel droit et l'axe A12 de l'orifice de raccord 12 à l'oreillette naturelle droite OD étant plus grand que l'angle C formé entre la direction générale 14 du ventricule artificiel gauche et l'axe A6 de l'orifice de raccord 6 à l'oreillette naturelle gauche OG. Dans l'exemple précédent où l'angle A est égal à approximativement 80°, les angles B et C peuvent être approximativement égaux à 50 et à 30 °, respectivement ; et
- les actionneurs hydrauliques individuels 7 et 1 3 sont disposés proches l'un de l'autre, du côté du ventricule artificiel gauche 2. Dans le mode de réalisation P de la prothèse selon l'invention (figure 3), les actionneurs hydrauliques individuels 7 et 13 sont disposés au voisinage de la pointe du V. Au contraire, dans le mode de réalisation P' (figure 5), lesdits actionneurs hydrauliques individuels 7 et 13 sont éloignés de cette pointe.

Ainsi, on obtient des prothèses P et P' compactes, aptes à se loger dans la cavité péricardique CP avec le ventricule artificiel droit 11 parallèle au thorax au droit du sternum, comme cela est illustré schématiquement sur la figure 4 pour la prothèse P. Eventuellement, comme également représenté sur la figure 4, l'encombrement de la prothèse selon l'invention peut être légèrement supérieur à la cavité péricardique CP (voir la ligne pointillée 16 délimitant intérieurement cette dernière). Dans ce cas, elle ne fait que comprimer légèrement le poumon gauche PG.

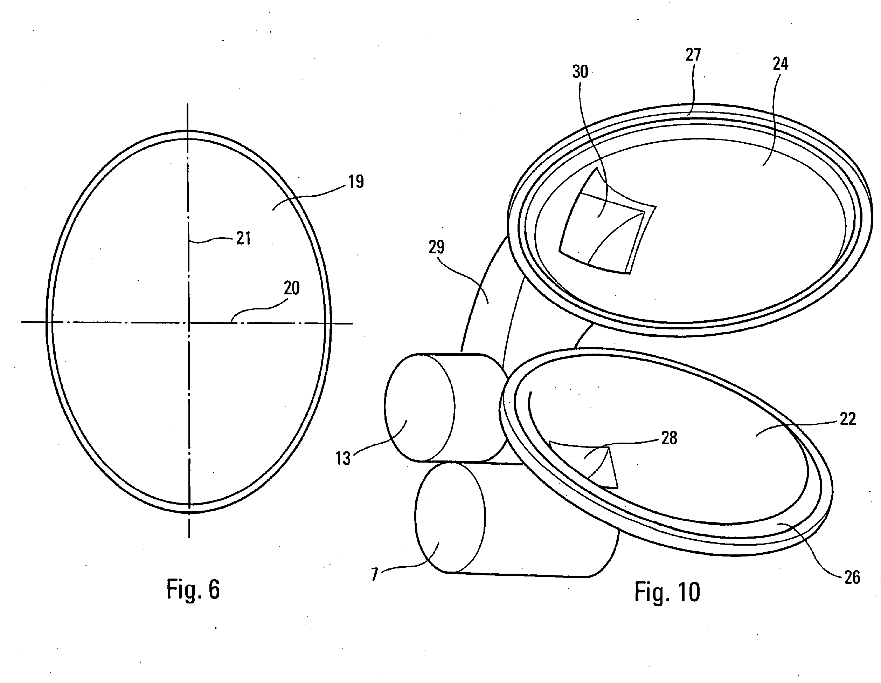

Par ailleurs, pour optimiser l'encombrement des prothèses cardiaques P et P' parallèlement à l'axe antéro-postérieur du thorax, il est avantageux que, vus en plan selon les flèches 17 et 18 des figures 3 et 5, les ventricules artificiels 2 et 8 présentent la forme d'une ellipse 19 (voir la figure 6), dont le petit axe 20 est parallèle à la direction générale 14 ou 15 et dont le grand axe 21 est transversal au plan de la figure 3. Chaque ventricule artificiel 2 et 8 est formé de deux dômes 22, 23 ou 24, 25 respectivement, opposés et reliés l'un à l'autre par une base 26, 27 présentant la forme de l'ellipse 19, en emprisonnant le bord de la membrane 3 ou 9. Pour un ventricule artificiel 2 ou 8 de capacité voisine de 70 cm³, le petit axe 20, le grand axe 21 et la distance d entre les sommets de deux dômes associés sont au moins approximativement égaux à 64 mm, 87 mm et 30 mm, respectivement.

Avantageusement, les dômes 23 et 25, correspondant respectivement aux chambres à sang 4 et 10, sont constitués par des évidements pratiqués dans le corps rigide 1, alors que les dômes 22 et 24, correspondant respectivement aux chambres à fluide hydraulique 5 et 11, sont conçus comme des couvercles venant obturer lesdits ventricules artificiels 2 et 8, respectivement (voir la figure 10).

L'actionneur hydraulique 7 étant proche du ventricule artificiel 2, sa sortie peut être reliée à ce dernier par le court passage 28 traversant le dôme 22. En revanche, l'actionneur hydraulique 13 étant éloigné du ventricule artificiel 8, sa sortie est reliée à ce dernier par le conduit 29 extérieur au corps 1, débouchant dans le dôme 24, à travers une ouverture 30 (voir la figure 10).

Comme le montrent les figures 7 et 8, les prothèses cardiaques P et P' se présentent sous la forme d'un volume de forme anatomique (correspondant à la forme de la cavité péricardique CP) pourvu d'un orifice 6 de raccord à l'oreillette naturelle gauche OG, d'un orifice 12 de raccord à l'oreillette naturelle droite OD, des moyens 33 de raccord à l'aorte AO et des moyens 32 de raccord à l'artère pulmonaire. De plus, sur ces figures, on a représenté la base 41 d'une connexion électrique avec l'extérieur.

Le corps 1 des prothèses cardiaques P et P' est enfermé de façon étanche dans un sac souple 34 entourant ledit corps, de façon ample, et étant rempli du fluide hydraulique actionné par les actionneurs 7 et 13, immergés dans ce fluide. Le sac souple 34 sert de plus de bâche pour ce fluide.

Entre le sac souple 34, d'une part, et le corps 1 et les actionneurs 7 et 13, d'autre part, est prévue une paroi rigide ajourée 35, faisant crépine et permettant la circulation du fluide hydraulique à l'intérieur du sac 34. La paroi ajourée 35 évite audit sac souple d'être aspiré par les actionneurs 7 et 13.

Entre la paroi rigide ajourée 35 et le sac 34 est ménagé un volume de débattement 40 pour ledit sac 34. De préférence, ce volume de débattement 40 est au moins approximativement égal au double du volume d'une chambre à fluide hydraulique 5, 11 d'un ventricule artificiel 2, 8.

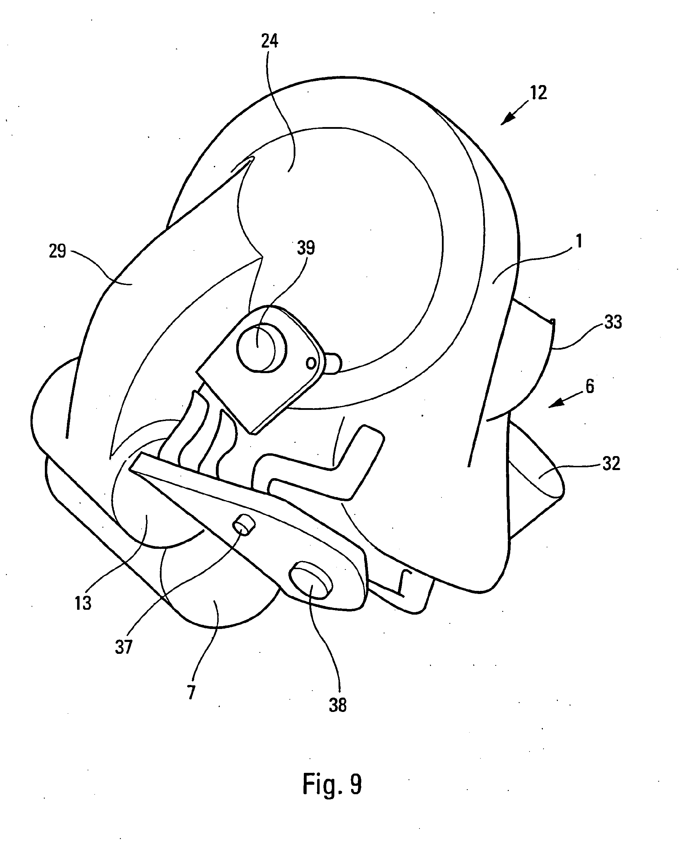

Sur la figure 9, on a représenté en perspective un mode de réalisation de l'ensemble du corps 1, des actionneurs 7 et 13 et du conduit 29 se trouvant à l'intérieur de la paroi enveloppante perforée 35 et du sac souple 34. Sur cette figure, les orifices 6 et 12 de raccord aux oreillettes naturelles gauche et droite OG et OD ne sont pas visibles, mais leurs positions sont indiquées par des flèches.

Sur cette figure 9, on a représenté de plus des éléments électroniques de pilotage, tels qu'un capteur 37 de la pression à l'intérieur du sac souple 34, un capteur 38 de la pression dans le ventricule artificiel gauche 2 et un capteur 39 de la pression dans le ventricule artificiel droit 8. Ainsi, la prothèse cardiaque conforme à la présente invention incorpore tous les éléments d'activation, de commande, de traitement, de communication et de régulation électroniques nécessaires à son fonctionnement, et qui, par conséquent, se trouvent logés en position anatomique dans la cavité péricardique.

## Revendications

1. Prothèse cardiaque implantable dans la cavité péricardique (CP) d'un patient, ladite prothèse étant apte à remplacer les ventricules gauche et droit naturels (VG, VD) dudit patient après ablation de ceux-ci et comportant un corps rigide (1) dans lequel sont agencés des ventricules gauche et droit artificiels (2, 8), chacun de ces ventricules artificiels comprenant une membrane souple (3, 9) :
- qui est apte à battre sous l'action d'un fluide hydraulique, et
- qui est disposée dans une cavité que ladite membrane partage de façon étanche en deux chambres (4, 5 - 10, 11) dont l'une est destinée à la circulation du sang et dont l'autre est emplie dudit fluide hydraulique,
les deux chambres à fluide hydraulique (5, 11) étant reliées à des actionneurs individuels hydrauliques (7, 13), tandis que la chambre à sang (4) du ventricule artificiel gauche (2) comporte un orifice de raccord (6) à l'oreillette naturelle gauche (OG) et des moyens (33) de raccord à l'aorte et que la chambre à sang (10) du ventricule artificiel droit (8) comporte un orifice de raccord (12) à l'oreillette naturelle droite (OD) et des moyens (32) de raccord à l'artère pulmonaire, les axes (A6, A12) desdits orifices (6, 12) de raccord aux oreillettes naturelles (OG, OD) étant coplanaires et lesdits ventricules artificiels (2, 8) présentant, parallèlement au plan desdits axes (A6, A12) desdits orifices (6, 12), des directions générales (14, 15) disposées en V, de façon que lesdits ventricules se rapprochent l'un de l'autre en s'éloignant desdits orifices (6, 12) de raccord aux oreillettes naturelles (OG, OD),
**caractérisée :**
- **en ce que** lesdits axes (A6, A 12) desdits orifices (6, 12) de raccord aux oreillettes naturelles (OG, OD) sont au moins approximativement parallèles ;
- **en ce que** ladite disposition en V des ventricules gauche et droit artificiels (2, 8) est asymétrique par rapport auxdits axes (A6, A12), l'angle (B) formé entre la direction générale (15) du ventricule artificiel droit (8) et l'axe (A12) dudit orifice de raccord (12) à l'oreillette naturelle droite (OD) étant plus grand que l'angle (C) formé entre la direction générale (14) du ventricule artificiel gauche (2) et l'axe (A6) dudit orifice de raccord (6) à l'oreillette naturelle gauche (OD) ; et
- **en ce que** lesdits actionneurs hydrauliques individuels (7, 13), associés respectivement auxdits ventricules gauche et droit artificiels (2, 8), sont disposés proches l'un de l'autre, du côté dudit ventricule artificiel gauche (2).

2. Prothèse cardiaque selon la revendication 1,
**caractérisée en ce que** la distance (E) entre lesdits axes parallèles (A6, A12) desdits orifices de raccord (6, 12) est au moins approximativement égale à 45 mm.

3. Prothèse cardiaque selon l'une des revendications 1 ou 2,
**caractérisée en ce que** lesdits actionneurs hydrauliques individuels (7, 13) sont disposés au voisinage de la pointe dudit V.

4. Prothèse cardiaque selon l'une des revendications 1 à 3,
**caractérisée en ce que** les dimensions (20) desdits ventricules artificiels (2, 8), parallèles auxdits directions générales (14, 15) de ceux-ci, sont plus petites que les dimensions (21) desdits ventricules perpendiculairement auxdites directions générales (14, 15).

5. Prothèse cardiaque selon la revendication 4, dans laquelle chacun desdits ventricules artificiels (2, 8) présente la forme de deux dômes opposés par rapport à une base commune,
**caractérisée en ce que** ladite base commune (26, 27) présente la forme d'une ellipse (19), dont le petit axe (20) est au moins sensiblement parallèle à ladite direction générale correspondante (14, 15) du ventricule.

6. Prothèse cardiaque selon la revendication 5 destinée à un adulte,
**caractérisée en ce que** les langueurs du petit axe (20) et du grand axe (21) de ladite base elliptique (19) sont au moins approximativement égales à 64 mm et 87 mm, respectivement.

7. Prothèse cardiaque selon la revendication 6,
**caractérisée en ce que** la distance (d) entre les sommets des deux dômes (22, 23 - 24, 25) est au moins approximativement égale à 30 mm.

8. Prothèse cardiaque selon l'une quelconque des revendications 1 à 7,
**caractérisée en ce que** l'angle (A) formé par lesdites directions générales (14, 15) desdits ventricules artificiels est au moins approximativement égal à 80°.

9. Prothèse cardiaque selon la revendication 8,
**caractérisé en ce que** l'angle (B) formé entre la direction générale (15) du ventricule artificiel droit (8) et l'axe (A 12) dudit orifice de raccord (12) à l'oreillette naturelle droite (OD) est au moins approximativement égal à 50° et **en ce que** l'angle (C) formé entre la direction générale (14) du ventricule artificiel gauche (2) et l'axe (A6) dudit orifice de raccord (6) à l'oreillette naturelle gauche (OG) est au moins approximativement égal à 30°.

10. Prothèse cardiaque selon l'une quelconque des revendications 1 à 9,
**caractérisée en ce que** l'actionneur hydraulique individuel (13) associé au ventricule droit artificiel (8) est relié à ce dernier par un conduit (29) extérieur audit corps rigide (1).

11. Prothèse cardiaque selon l'une quelconque des revendications 1 à 10,
**caractérisée en ce qu'**elle comporte :
- un sac souple (34) entourant, de façon ample et étanche, au moins une partie dudit corps rigide (1) en enfermant lesdits actionneurs hydrauliques (7, 13) et l'électronique de commande, de traitement des signaux et de communication, ledit sac étant rempli dudit fluide hydraulique et servant de bâche au circuit hydraulique desdits actionneurs ; et
- une paroi rigide ajourée enveloppante (35), solidaire dudit corps rigide (1) et disposée entre celui-ci et ledit sac souple (34).

12. Prothèse cardiaque selon la revendication 11,
**caractérisée en ce qu'**un volume de débattement (40) pour ledit sac souple (34) est ménagé entre celui-ci et ladite paroi rigide ajourée (35).

13. Prothèse cardiaque selon la revendication 12,
**caractérisée en ce que** ledit volume de débattement (40) est au moins approximativement égal au double du volume de la chambre à fluide hydraulique d'un desdits ventricules artificiels (2, 8) et est réparti sur toute la surface de la paroi rigide.

## Claims

1. Heart prosthesis implantable in the pericardial cavity (CP) of a patient, said prosthesis being able to replace the natural left and right ventricles (VG, VD) of said patient after their removal and comprising a rigid body (1) in which artificial left and right ventricles (2, 8) are arranged, each of these artificial ventricles comprising a flexible membrane (3, 9):
- which is able to beat under the action of a hydraulic fluid, and
- which is arranged in a cavity divided in a leaktight manner by said membrane into two chambers (4, 5 - 10, 11), one of which is intended for the circulation of the blood, and the other of which is filled with said hydraulic fluid,
the two hydraulic fluid chambers (5, 11) being connected to individual hydraulic actuators (7, 13), while the blood chamber (4) of the artificial left ventricle (2) comprises an orifice (6) of connection to the natural left auricle (OG) and means (33) of connection to the aorta, and the blood chamber (10) of the artificial right ventricle (8) comprises an orifice (12) of connection to the natural right auricle (OD) and means (32) of connection to the pulmonary artery, the axes (A6, A12) of said orifices (6, 12) of connection to the natural auricles (OG, OD) being co-planar, and said artificial ventricles (2, 8) having, parallel to the plane of said axes (A6, A12) of said orifices (6, 12), general directions (14, 15) arranged in a V-shape, such that said ventricles approach each other as they move away from said orifices (6, 12) of connection to the natural auricles (OG, OD),
**characterized:**
- **in that** said axes (A6, A12) of said orifices (6, 12) of connection to the natural auricles (OG, OD) are at least approximately parallel;
- **in that** said V-shaped arrangement of the artificial left and right ventricles (2, 8) is asymmetrical with respect to said axes (A6, A12), the angle (B) formed between the general direction (15) of the artificial right ventricle (8) and the axis (A12) of said orifice (12) of connection to the natural right auricle (OD) being greater than the angle (C) formed between the general direction (14) of the artificial left ventricle (2) and the axis (A6) of said orifice (6) of connection to the natural left auricle (OG); and
- **in that** said individual hydraulic actuators (7, 13), associated respectively with said artificial left and right ventricles (2, 8), are arranged near each other, on the side of said artificial left ventricle (2).

2. Heart prosthesis according to Claim 1, **characterized in that** the distance (E) between said parallel axes (A6, A12) of said connection orifices (6, 12) is at least approximately equal to 45 mm.

3. Heart prosthesis according to one of Claims 1 or 2, **characterized in that** said individual hydraulic actuators (7, 13) are arranged in proximity to the tip of said V.

4. Heart prosthesis according to one of Claims 1 to 3, **characterized in that** the dimensions (20) of said artificial ventricles (2, 8), parallel to said general directions (14, 15) thereof, are smaller than the dimensions (21) of said ventricles perpendicular to said general directions (14, 15).

5. Heart prosthesis according to Claim 4, in which each of said artificial ventricles (2, 8) has the shape of two domes arranged opposite with respect to a common base, **characterized in that** said common base (26, 27) has the shape of an ellipse (19), of which the minor axis (20) is at least substantially parallel to said corresponding general direction (14, 15) of the ventricle.

6. Heart prosthesis according to Claim 5 intended for an adult, **characterized in that** the lengths of the minor axis (20) and of the major axis (21) of said elliptic base (19) are at least approximately equal to 64 mm and 87 mm, respectively.

7. Heart prosthesis according to Claim 6, **characterized in that** the distance (d) between the summits of the two domes (22, 23 - 24, 25) is at least approximately equal to 30 mm.

8. Heart prosthesis according to any one of Claims 1 to 7, **characterized in that** the angle (A) formed by said general directions (14, 15) of said artificial ventricles is at least approximately equal to 80°.

9. Heart prosthesis according to Claim 8, **characterized in that** the angle (B) formed between the general direction (15) of the artificial right ventricle (8) and the axis (A12) of said orifice (12) of connection to the natural right auricle (RA) is at least approximately equal to 50°, and **in that** the angle (C) formed between the general direction (14) of the artificial left ventricle (2) and the axis (A6) of said orifice (6) of connection to the natural left auricle (LA) is at least approximately equal to 30°.

10. Heart prosthesis according to any one of Claims 1 to 9, **characterized in that** the individual hydraulic actuator (13) associated with the artificial right ventricle (8) is connected to the latter via a passage (29) outside said rigid body (1).

11. Heart prosthesis according to any one of Claims 1 to 10, **characterized in that** it comprises:
- a flexible pouch (34) surrounding, amply and sealingly, at least part of said rigid body (1) by enclosing said hydraulic actuators (7, 13) and the electronics for control, signal processing and communication, said pouch being filled with said hydraulic fluid and serving as a container for the hydraulic circuit of said actuators; and
- a surrounding openworked and rigid wall (35) which is integral with said rigid body (1) and which is arranged between the latter and said flexible pouch (34).

12. Heart prosthesis according to Claim 11, **characterized in that** a clearance volume (40) for said flexible pouch (34) is formed between it and said openworked rigid wall (35).

13. Heart prosthesis according to Claim 12, **characterized in that** said clearance volume (40) is at least approximately equal to twice the volume of the hydraulic fluid chamber of one of said artificial ventricles (2, 8) and is distributed across the full surface of the rigid wall.

## Patentansprüche

1. In die Perikardhöhle (CP) eines Patienten implantierbare Herzprothese, wobei die Prothese geeignet ist, die natürliche linke und rechte Herzkammer (VG, VD) des Patienten nach deren Ablation zu ersetzen, und wobei sie einen starren Körper (1) umfasst, in dem eine künstliche linke und rechte Herzkammer (2, 8) gebildet sind, wobei jede dieser künstlichen Herzkammern eine flexible Membran (3, 9) umfasst,
- die geeignet ist, unter der Einwirkung eines Hydraulik-Fluid zu schlagen, und
- die in einem Hohlraum angeordnet ist, den die Membran auf dichte Weise in zwei Kammern (4, 5 - 10, 11) unterteilt, wobei die eine zur Zirkulation des Bluts bestimmt ist und wobei die andere mit dem Hydraulik-Fluid gefüllt ist,
wobei die beiden Hydraulikfluidkammern (5, 11) mit individuellen hydraulischen Betätigungsvorrichtungen (7, 13) verbunden sind, während die Blutkammer (4) der künstlichen linken Herzkammer (2) eine Öffnung zum Anschluss (6) an das natürliche linke Herzohr (OG) und Mittel (33) zum Anschluss an die Aorta umfasst und während die Blutkammer (10) der künstlichen rechten Herzkammer (8) eine Öffnung zum Anschluss (12) an das natürliche rechte Herzohr (OD) und Mittel (32) zum Anschluss an die Lungenarterie umfasst, wobei die Achsen (A6, A12) der Öffnungen (6, 12) zum Anschluss an die natürlichen Herzohren (OG, OD) koplanar sind und die künstlichen Herzkammern (2, 8) parallel zur Ebene der Achsen (A6, A12) der Öffnungen (6, 12) allgemeine Richtungen (14, 15) aufweisen, die V-förmig angeordnet sind, so dass sich die Herzkammern aneinander annähern, indem sie sich von den Öffnungen (6, 12) zum Anschluss an die natürlichen Herzohren (OG, OD) entfernen,
**dadurch gekennzeichnet,**
- **dass** die Achsen (A6, A12) der Öffnungen (6, 12) zum Anschluss an die natürlichen Herzohren (OG, OD) mindestens annähernd parallel sind;
- **dass** die V-förmige Anordnung der künstlichen linken und rechten Herzkammer (2, 8) in Bezug auf die Achsen (A6, A12) asymmetrisch ist, wobei der Winkel (B), der zwischen der allgemeinen Richtung (15) der künstlichen rechten Herzkammer (8) und der Achse (A12) der Öffnung zum Anschluss (12) an das natürliche rechte Herzohr (OD) gebildet ist, größer ist als der Winkel (C), der zwischen der allgemeinen Richtung (14) der künstlichen linken Herzkammer (2) und der Achse (A6) der Öffnung zum Anschluss (6) an das natürliche linke Herzohr (OG) gebildet ist; und
- **dass** die individuellen hydraulischen Betätigungsvorrichtungen (7, 13), die der künstlichen linken bzw. rechten Herzkammer (2, 8) zugeordnet sind, nahe beieinander an der Seite der künstlichen linken Herzkammer (2) angeordnet sind.

2. Herzprothese nach Anspruch 1,
**dadurch gekennzeichnet, dass** der Abstand (E) zwischen den parallelen Achsen (A6, A12) der Anschlussöffnungen (6, 12) mindestens annähernd gleich 45 mm ist.

3. Herzprothese nach einem der Ansprüche 1 oder 2,
**dadurch gekennzeichnet, dass** die individuellen hydraulischen Betätigungsvorrichtungen (7, 13) in der Nähe der Spitze des V angeordnet sind.

4. Herzprothese nach einem der Ansprüche 1 bis 3,
**dadurch gekennzeichnet, dass** die Abmessungen (20) der künstlichen Herzkammern (2, 8) parallel zu deren allgemeinen Richtungen (14, 15) kleiner sind als die Abmessungen (21) der Herzkammern senkrecht zu den allgemeinen Richtungen (14, 15).

5. Herzprothese nach Anspruch 4, wobei jede der künstlichen Herzkammern (2, 8) die Form von zwei Kuppeln aufweist, die in Bezug auf eine gemeinsame Basis einander gegenüberliegen,
**dadurch gekennzeichnet, dass** die gemeinsame Basis (26, 27) die Form einer Ellipse (19) aufweist, deren kleine Achse (20) mindestens im Wesentlichen parallel zur entsprechenden allgemeinen Richtung (14, 15) der Herzkammer ist.

6. Herzprothese nach Anspruch 5, welche für einen Erwachsenen bestimmt ist,
**dadurch gekennzeichnet, dass** die Längen der kleinen Achse (20) und der großen Achse (21) der elliptischen Basis (19) mindestens annähernd gleich 64 mm bzw. 87 mm sind.

7. Herzprothese nach Anspruch 6,
**dadurch gekennzeichnet, dass** der Abstand (d) zwischen den Scheiteln der beiden Kuppeln (22, 23 - 24, 25) mindestens annähernd gleich 30 mm ist.

8. Herzprothese nach einem der Ansprüche 1 bis 7,
**dadurch gekennzeichnet, dass** der Winkel (A), der durch die allgemeinen Richtungen (14, 15) der künstlichen Herzkammern gebildet wird, mindestens annähernd gleich 80° ist.

9. Herzprothese nach Anspruch 8,
**dadurch gekennzeichnet, dass** der Winkel (B), der zwischen der allgemeinen Richtung (15) der künstlichen rechten Herzkammer (8) und der Achse (A12) der Öffnung zum Anschluss (12) an das natürliche rechte Herzohr (OD) gebildet wird, mindestens annähernd gleich 50° ist und dass der Winkel (C), der zwischen der allgemeinen Richtung (14) der künstlichen linken Herzkammer (2) und der Achse (A6) der Öffnung zum Anschluss (6) an das natürliche linke Herzohr (OG) gebildet wird, mindestens annähernd gleich 30° ist.

10. Herzprothese nach einem der Ansprüche 1 bis 9,
**dadurch gekennzeichnet, dass** die individuelle hydraulische Betätigungsvorrichtung (13), die der künstlichen rechten Herzkammer (8) zugeordnet ist, mit dieser durch einen Kanal (29) verbunden ist, der außerhalb des starren Körpers (1) angeordnet ist.

11. Herzprothese nach einem der Ansprüche 1 bis 10,
**dadurch gekennzeichnet, dass** sie umfasst:
- einen flexiblen Beutel (34), der auf weite und dichte Weise mindestens einen Teil des starren Körpers (1) umgibt, indem er die hydraulischen Betätigungsvorrichtungen (7, 13) und die Elektronik zur Steuerung, Signalverarbeitung und Kommunikation einschließt, wobei der Beutel mit dem Hydraulik-Fluid gefüllt ist und als Gehäuse für den Hydraulikkreis der Betätigungsvorrichtungen dient; und
- eine umgebende durchbrochene starre Wand (35), die mit dem starren Körper (1) fest verbunden ist und zwischen diesem und dem flexiblen Beutel (34) angeordnet ist.

12. Herzprothese nach Anspruch 11,
**dadurch gekennzeichnet, dass** ein Ausfederungsvolumen (40) für den flexiblen Beutel (34) zwischen diesem und der durchbrochenen starren Wand (35) angeordnet ist.

13. Herzprothese nach Anspruch 12,
**dadurch gekennzeichnet, dass** das Ausfederungsvolumen (40) mindestens annähernd gleich dem Doppelten des Volumens der Hydraulikfluidkammer einer der künstlichen Herzkammern (2, 8) ist und über die gesamte Oberfläche der starren Wand verteilt ist.
